(19) Europäisches Patentamt / European Patent Office / Office européen des brevets

(11) **EP 4 427 759 A1**

(12) **EUROPEAN PATENT APPLICATION**

(43) Date of publication:
**11.09.2024 Bulletin 2024/37**

(21) Application number: **23161072.6**

(22) Date of filing: **09.03.2023**

(51) International Patent Classification (IPC):
**A61K 39/00** *(2006.01)*     **C07K 16/00** *(2006.01)*

(52) Cooperative Patent Classification (CPC):
**A61K 39/4631; A61K 39/4611; A61K 39/4644;
C07K 16/00; G01N 33/5091; G01N 33/534;**
A61K 2239/24; A61K 2239/48; A61K 2239/50

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Eberhard-Karls-Universität Tübingen
72076 Tübingen (DE)**

(72) Inventors:
• **Stotz, Sophie
  72074 Tübingen (DE)**
• **Maurer, Andreas
  72074 Tübingen (DE)**

• **Schlegel, Patrick
  2153 Bella Vista (DE)**
• **Seitz, Christian Martin
  72072 Tübingen (DE)**
• **Pichler, Bernd
  85298 Fernhag (DE)**
• **Stremme, Ramona
  72076 Tübingen (DE)**
• **Haupt, Dennis Christopher Ulrich
  72076 Tübingen (DE)**

(74) Representative: **Witte, Weller & Partner
Patentanwälte mbB
Postfach 10 54 62
70047 Stuttgart (DE)**

(54) **TRACEABLE CHIMERIC ANTIGEN RECEPTOR CELL**

(57)     The invention relates to a composition comprising a chimeric antigen receptor cell (CAR cell) whose distribution in the organism can be easily monitored and tracked in subjects using conventional imaging techniques. The invention further relates to a method of treatment of a living being in need for a CAR cell therapy.

**Description**

FIELD OF THE INVENTION

The invention relates to a composition comprising a chimeric antigen receptor cell (CAR cell) whose distribution in the organism can be easily monitored and tracked in subjects using conventional imaging techniques. The invention further relates to a method of treatment of a living being in need for a CAR cell therapy.

BACKGROUND OF THE INVENTION

Today, cancer is still one of the deadliest diseases of humankind. Over the past decades huge efforts have been made to assist the own immune system in fighting cancer.

In theory, the immune system can detect malignant cells and even remove them on its own. Still, some of those malignant cells can avoid detection or even suppress the reaction of the immune system, which renders their elimination from the body difficult.

There are two interconnected layers of immunity. In innate immunity an unspecific, fast response is initiated. An example is phagocytosis, which is performed by macrophages, where the cell engulfs large particles or pathogen cells with its cell membrane. In combination with the adaptive immunity, the innate immune system fights against exogenous invaders and endogenously caused hazards with the goal to eliminate those pathogens. The adaptive immune system is comparably slower than the innate immunity due to its specificity that relies on the proliferation of specialized cell populations. Looking more closely at the adaptive immune system there are two major mechanisms involved: One being the cell-mediated response whereas the other is the humoral response.

Both responses are mediated through lymphocytes, namely T cells and B cells, which originate from the multipotential hematopoietic stem cell line. At first, the cells are produced in primary lymphatic organs and circulate in the blood as naive cells. Upon contact with their respective antigen they are primed and turned into effector cells, which now can react against potential pathogens. Upon binding to the pathogen, downstream signaling occurs, leading to killing of the target cell. Once the adaptive immune response is over, T cells are reduced to maintain the metabolic cost. Alternatively, a conversion into long-lasting memory cells is also possible. Memory cells can re-activate if an infection with the same pathogen is present.

The T cells originate from hematopoietic stem cells located in the bone marrow. Several differentiation steps occur after their migration into the thymus, which ultimately turns them into effector cells. This differentiation includes a rearrangement of the T cell receptor (TCR) turning them into double positive $CD4^+$ and $CD8^+$ (DP) thymocytes. CD4 or CD8 are receptors located on the outer membrane of T cells, responsible for T cell interaction with major histocompatibility complex (MHC) molecules type I or II. Firstly, the double negative T cell creates the alpha and beta chain of the $CD4^+$ and $CD8^+$ TCR. The presentation of self-antigens on the thymic cortical epithelial cells on MHC molecules leads to a double positive selection. The DP cells are only an intermediate state and require further differentiation to create either $CD4^+$ or $CD8^+$ single positives (SP). Only cells with a strong affinity towards MHC molecules will undergo proliferation or, otherwise, apoptosis is initiated. Furthermore, selection of single positive cells takes place, where cells with strong affinity towards MHCI show an upregulation of CD8 marker (T killer cells), whereas cells with strong affinity towards MHCII show an upregulation of CD4 (T-helper cells).

The TCR contains an alpha and beta chain, each with a variable and constant region. Additionally, these chains possess three hypervariable regions, which are responsible for the diversity of antigen specificity. The TCR can bind to a variety of different antigens due to the genetic recombination of those regions, also called VDJ-recom bi nation.

After the selection, the T cells are released into the blood stream. For T cell activation, the antigen presenting cells (APCs) need to present peptides from pathogens on their surface, occurring due to internal degradation of exogenous (MHC class II) or endogenous (MHC class I) proteins. APC bind with their MHC-peptide complex to the TCR-CD3 complex, whereby CD3 is a protein necessary for T cell activation. Additionally, the CD4 or CD8 receptor binds to the MHC class II or I molecule, respectively, to further stabilize the complex. The T cell surface also includes a CD28 receptor, which is responsible for co-stimulation, an important step to induce proliferation. After proliferation, if the T cell encounters a cell presenting an antigen fitting to its T cell receptor it can induce apoptosis within the target cell with the help of perforin, granzymes or the FAS-receptor.

One goal of immunotherapy is to develop cancer-targeting therapies. This requires detailed knowledge about the molecular mechanisms and progression. Research of the last decade has revealed the unique molecular patterns for cancerous cells, also called 'hallmarks of cancer', that can be targeted using such therapies.

Cancerous cells have the possibility to produce growth factor ligands themselves (mitogenic signaling), which grants the capability to sustain chronic proliferation. Moreover, autocrine proliferation is possible as a result of the expression of associated receptors like vascular endothelial growth factor (VEGF).

Another aspect is the resistance towards tumor suppressors. The regulation of the cell proliferation is caused by proteins such as TP53 (also known as p53), leading to a stop in cell division, and induction of senescence. If DNA damage is present, apoptosis follows. 19 Infinite cell division can be achieved if a point mutation is present in the p53 protein, rendering it non-functional. Additionally, the inactivation of pro-apoptotic proteins, which are necessary for downstream activation of caspases, is induced by suppressive proteins like Bcl-2. In healthy cells, the downstream activation leads to cell death.

This almost limitless replication aligns with the elongation of telomers, protecting the chromosome. The cell is protected from senescence and apoptosis by a combination of telomerase overexpression and inhibition of telomer shortening.

Furthermore, the tumor can form new blood vessels in proximity to its microenvironment, the so-called neoangiogenesis. It is induced by e.g., vascular endothelial growth factor-A (VEGF-A) and thrombospondin-1 (TSP-1). The neoangiogenesis will provide oxygen and nutrients to the malignant cells.

Lastly, the formation of metastases, the invasion of cancer cells into various organs due to modification of proteins responsible for the connection between cells and the environment, contributes immensely to the bad prognosis of many cancers.

The T cells of the adaptive immune system can detect the presence of cancerous cells with the help of tumor associated antigens (TAAs). Those are peptide fragments presented on the surface of MHC molecules and are derived from the cancer cell peptidome.

However, malignant cells can evade the immune system and therefore the treatment of cancer presents itself as a difficult challenge. One example of an evasion mechanism is the alteration of the processing and presentation of antigens on MHC molecules of the tumor cells. This would lead to an inhibition of the T cell response. To bypass this mechanism, immunotherapy is a valuable tool.

Using immunotherapy, the body's immune system is either activated or suppressed. If immunotherapy is used to stimulate the immune system or amplify immune cells, it is called activation immunotherapy, with the opposite being suppression immunotherapy. In the case of treating cancer, activation immunotherapy is used. Up to this day, only few immunotherapies are approved for cancer patients. One being the use of interferon alpha against melanoma or lymphoma. Interferon alpha is a cytokine enhancing the proliferation of B cells and natural killer cells. Another example is the use of thalidomide against multiple myeloma. It prevents angiogenesis and increases the proliferation and activation of some immune cells.

Activation therapy can redirect the immune system directly onto cancerous cells and thereby bypass the evasion mechanism of some tumor entities. This is accomplished by targeting suitable structures on the tumor surface. However, since cancer is usually very heterogenous, not all established therapies work for every patient.

T cell proliferation takes quite a long time. Even longer to acquire enough T cells with the right receptor against the target tumor surface marker. Artificially supplying T cell with an additional tailor-made receptor that directly targets tumor antigens allows the rapid and effective generation of a T cell population for cancer immunotherapy. Such receptors are typically generated as a chimera of an antibody-derived binding protein and the signaling domains of the T cell receptor, so-called chimeric antigen receptors (CAR). This particular technique to extend the specificity of T cells has been particularly successful for non-solid tumors and is thus already approved for treating B cell malignancies and lymphoma.

The recognition of intact cell surface proteins is performed by the single-chain fragment (scFv) through which antigen processing and the MHC pathway is no longer needed. The chimeric antigen receptors (CARs) are proteins engineered of multiple segments which are fused together. There are three different generations of CARs. The generation 1 CAR consists only of an extracellular binding domain derived from the scFv of an antibody against a surface marker, which is linked with a spacer peptide to a transmembrane domain e.g., CD3$\zeta$. CD3$\zeta$ is a receptor necessary for T cell activation due to intracellular signaling and by adding one or two costimulatory factors a CAR T cell of $2^{nd}$ or $3^{rd}$ generation can be generated. An example is CD28, which mimics co-stimulation provided during TCR recognition and is mandatory for complete T cell activation. CAR T cells of higher generation also possess higher proliferation and cytokine production due to said costimulatory factors. The key to generate CAR expressing T cells or CAR T cells is gene transfer technology. This independency also creates an insensitivity towards the escape mechanisms related to MHC loss, making it superior to the regular activation immunotherapy. Since CAR T cells are directed against the tumor surface markers, they also reduce off target effects. CAR T cells are predominantly used for B cell malignances as lymphoma and multiple myeloma. Since CAR T cell penetration into solid tumors is low, they are up until now not effective enough to treat these entities.

Leukapheresis is one method to harvest autologous T cells for the generation of CAR T cells for the use in patients. The blood of the patient is collected and filtered, whereby the excess leukocytes are separated from the remaining blood. Redundant components like myeloid cells are washed out. The now enriched T cells are activated through paramagnetic beads and are then transduced with a viral vector encoding for the CAR transgene. To increase the number of cells they are cultivated *in vitro* and ultimately harvested to obtain the final CAR T cell product, which is infused back into the patient. Their task inside the patient is to detect surface antigens of cancerous cells with the help of the CAR. A successful binding to the antigen will result in killing of the target cell.

However, despite all selection steps there is still the possibility of myeloid cells (monocytes, neutrophils and dendritic cells), natural killer cells (NKs), erythroid cells as well as untransduced T cells being present, making it a heterogenous population. Those remains can show inhibitory effects, decrease the expansion of CAR T cells and ultimately decrease efficiency. An already approved CAR T cell therapy is provided by Novartis and is applied under the name Kymriah (Tisagenlecleucel). This therapy yields a high success rate for the treated types of cancer like diffuse large B cell lymphoma (DLBCL) and acute lymphoblastic leukemia (ALL), by targeting the CD19 surface protein.

Even though CAR T cells are a great start towards cancer immunotherapy they also come with a downside. E.g., in solid tumors, CAR T cell migration and function is limited due to physical barriers and immunosuppressive microenvironment. "On-target, off-tumor" toxicities can occur generated from low levels of target antigen expression in healthy tissues, but cytokine release syndrome (CRS) can also be a possible side effect. To overcome the afore mentioned limitations, adapter chimeric antigen receptors (AdCAR) were developed. The intention behind AdCAR is to improve flexibility, tumor specificity as well as controllability of conventional CAR T cells. The conventional CAR system was uncoupled, creating a dichotomous system.

An overview on the adapter chimeric antigen receptor T cells is given by Arndt, C. et al. Adaptor CAR Platforms - Next Generation of T Cell-Based Cancer Immunotherapy. Cancers, Vol. 12, No. 1302 (2020).

Due to their dichotomous nature, adaptor CAR cells overcome the rigid mono-specificity of conventional CAR cells. This flexibility offers novel possibilities to encounter one of the central problems of conventional CAR T cell therapy - the antigen-negative relapse. One adaptor CAR cell can redirect T cells against a theoretically unlimited number of target antigens. Thus, the technology allows the manufacturing of one bioengineered T cell product universally applicable for all types of cancer. This obviates the need for the laborious and cost-intensive development of new CAR constructs and genetically modified immune cells. Provided that a comprehensive library of appropriate adaptor molecules is available, adaptor CAR T cells can be easily used for simultaneous or consecutive multiple tumor targeting.

The adapter molecule consists of two parts, with one being an antibody against tumor specific surface markers and the other one a moiety enabling engagement of the AdCAR (e.g., biotin, avidin or FITC); see WO 2018/078066 and Seitz et al. Novel adapter CAR-T cell technology for precisely controllable multiplex cancer targeting. Oncoimmunology, Vol. 10, No. 1, e2003532 (2021).

One advantage of the adapter system over conventional CAR T cells is the reduction or even shutdown of anti-tumor responses when supply with the adaptor molecule is stopped. Moreover, it is possible to re-initiate the therapy against the same or even different targets. So, one AdCAR T cell can detect and bind an infinite number of antigens with the help of different adaptor molecules. Since only one CAR T cell product is necessary, costs and effort can be reduced immensely.

The use of cell therapies is a relatively new way of therapy. Therefore, it is indispensable to gain sufficient knowledge of their distribution *in vivo.* The difficulties to predict toxicities as well as therapeutical success or failure are a further motivation to track T cell transfer therapies.

Non-invasive imaging is a powerful complementary tool to image immune cells. Computerized tomography (CT) and magnetic resonance imaging (MRI) give excellent visualization of the anatomy. While CT is mainly used for dense structures like bones, MRI has a better soft tissue contrast.

Since those methods only give anatomical but not functional information, positron emission tomography (PET) is used for e.g., molecular interactions within the body or characterization of tumor entities. To create an image, small molecules, peptides, or biologicals are labeled with positron emitting radionuclides and are injected into the organism. *In vivo,* the instable nucleus emits a positron travelling an isotope-dependent distance into the surrounding tissue, where it annihilates when meeting an electron. Scintillator crystals around the subject can now detect the two opposed $\gamma$-photons created by the annihilation process. Those events are then back-calculated to determine the exact position of radioactive decay.

One possible way of tracking T cells *in vivo* with PET is the use of a reporter gene system. One example of a reporter gene system is the sodium-iodine-symporter (NIS). This symporter is expressed in the thyroid and some other tissues, facilitating the transport of iodine. For PET studies, cells transfected with the NIS gene can be monitored by using radiotracers such as [124I]iodide or [18F]tetraflouroborate. However, this comes with some limitations. As NIS is also expressed in normal cells, organs with NIS expressing cells (the thyroid) exhibit high background signal.

Another prominent reporter gene is the herpes simplex virus type 1 thymidine kinase (HSV1-tk) with its radiotracer [18F]-9-(4-fluoro-3-hydroxymethylbutyl)guanine ([18F]FHBG). This gene encodes for an intracellular protein and thus the risk of an immune response is reduced. As anti-viral agents are specific for the virus kinase and therefore not recognized by the human kinase, the background signal is low. Upon entering the cell, FHBG undergoes a phosphorylation step to then remain trapped inside the cell. This reporter gene system has already achieved promising results in patients with glioblastoma.

Recently, SNAPTag proved to be a reliable reporter gene for PET studies as well. The reporter gene is originally derived from O6-methylguanine methyl transferase (MGMT), which is considered a vital DNA repair enzyme that dealkylates O6-methylguanine in an irreversible manner. The big advantage of the SNAP-tag is its small size, which makes it easy to incorporate into gene delivery vectors. Additionally, it is not expressed endogenously and thus has low off-target binding. Radiotracers targeting SNAPTag have been successfully evaluated in murine xenograft models but have not been tested for T cell tracking.

There is also the possibility of *in vivo* cell imaging with MRI. One possibility is with the help of the transferrin receptor to capture transferrin-conjugated superparamagnetic iron oxide (SPIO) particles. So, cells transfected with the ferritin gene show increased uptake of iron, which is detectable by MRI. However, reporter systems are often not sensitive enough.

In general, reporter gene systems have a variety of disadvantages. Firstly, the genes are relatively big. Therefore, vector capacity must also be considered. Moreover, there is the risk of endogenous reporter genes being expressed in normal cells as well, leading to high background uptake (e.g., NIS). Exogenous reporters carry the risk of immunogenicity and could cause a possibly dangerous immune reaction.

It is immensely important to control whether the CAR T cells also arrive at the tumor or accumulate in unwanted organs (e.g., spleen). However, *in vivo* tracking of CAR T cells is still a major challenge in preclinical and clinical research; see Marofi, F. et al. CAR T cells in solid tumors: challenges and opportunities. Stem Cell Res Ther Vol. 12, No. 81 (2021). Visualization of CAR T cells is of immense importance for the development of new immunotherapies against cancers and helps to better understand and ultimately treat solid tumors; see Kim, W. et al. In vivo tracking of bioorthogonally labeled T-cells for predicting therapeutic efficacy of adoptive T-cell therapy. J. Control. Release, Vol. 329, pp. 223-236, (2021). Previous approaches introduce another transgene or reporter into AdCAR T cells, but this is laborious and may affect the T cells; see Sellmyer, M. A. et al. Imaging CAR T Cell Trafficking with eDHFR as a PET Reporter Gene. Mol. Ther., Vol. 28, pp. 42-51 (2020).

Against this background, it is the problem of the present invention to provide a possibility to track CAR cells, *in vivo,* in particular to be able to better evaluate the biodistribution, i.e., the distribution in the body, and thereby to avoid or at least reduce the disadvantages of the prior art tracking methods. Thus, the introduction of another transgene or reporter into the CAR cells and thus a negative influence on the cells and the immense amount of work in the art should be avoided.

SUMMARY OF THE INVENTION

This problem is solved by the provision of a composition comprising (i) a chimeric antigen receptor cell (CAR cell), and (ii) a radio-labelled ligand for the chimeric antigen receptor (CAR) of the CAR cell.

The inventors have realized that, surprisingly, the therapeutic function of the chimeric antigen receptor or CAR can also be used diagnostically or for tracking the CAR cells, so that the additional introduction of further transgenes or reporter genes into the CAR cell can be dispensed with. The effort required for this is consequently eliminated. There is also no risk that the introduction of additional transgenes or reporter genes will influence the function of the CAR cell.

According to the invention, a "CAR cell" can be any cell suitable for and capable of properly expressing the chimeric antigen receptor (CAR), such as an immune cell, in particular a T cell (CAR T cell), a natural killer cell (CAR NK cell), a T regulatory cell (CAR Treg cell), or a stem cell (CAR stem cell).

The structure of a CAR cell is well known to the skilled person. In general, a CAR may comprise an extracellular domain (extracellular part) comprising the antigen binding domain, a transmembrane domain and an intracellular signaling domain. The extracellular domain may be linked to the transmembrane domain by a linker. The extracellular domain may also comprise a signal peptide. Generally, an "antigen binding domain" refers to the region of the CAR that specifically binds to an antigen (and thereby is able to target a cell containing the antigen). The CARs of the invention may comprise one or more antigen binding domains. The antigen binding domain may comprise an antibody or an antigen binding fragment thereof. The antigen binding domain may comprise, for example, full length heavy chain, Fab fragments, single chain Fv (scFv) fragments, divalent single chain antibodies or diabodies. Any molecule that binds specifically to a given antigen such as affibodies or ligand binding domains from naturally occurring receptors may be used as an antigen binding domain. Often the antigen binding domain is a scFv. Normally, in a scFv the variable regions of an immunoglobulin heavy chain and light chain are fused by a flexible linker to form a scFv. Such a linker may be for example the "(G4/S1)3-linker".

According to the invention, a "ligand" for the CAR is understood to be a molecule which recognizes and binds to the antigen-binding domain of the extracellular domain of the CAR in a specific manner. "Specific" means that the molecule does not substantially recognize or bind other structures in a sample. Examples of a ligand include, without being restricted thereto, any antigenic structure of interest in a CAR therapy, such as CD19, CD22, BCMA, GD2, etc. They also include molecules that are suitable as moieties of adapter molecules in AdCAR platforms, such as biotin, avidin, fluorescein isothiocyanate (FITC), etc.

According to the invention, the ligand for the CAR is "radio-labelled". Radio-labeled means, according to the invention, that the ligand comprises a radioactive marker, such as, for example, a radioisotope coupled to it, e.g., $^{18}$F, $^{64}$Cu, $^{68}$Ga, $^{99m}$Tc, etc.

This means that the radio-labeled ligand for the CAR can be, according to the invention, in the case of using a conventional CAR cell platform, for example, a radio-labeled mimetic peptide of CD19, CD22, etc., or, if an AdCAR cell platform is used, any radio labelled adapter molecule.

The composition according to the invention now allows - via the traceability of the CAR cells in the organism or tissue - for the very first time to better understand whether the CAR cells also arrive at the target structure, e.g., the tumor, or, if necessary, accumulate in undesired organs, e.g., the spleen.

The problem underlying the invention is herewith fully achieved.

In an embodiment of the invention (ii) is a radio-labelled ligand for the antigen binding site of the CAR of the CAR cell.

The inventors have recognized that the antigen binding site of the CAR in particular can be advantageously radio-labeled, or preferably a radio-labeled ligand can be used for the antigen binding site of the CAR.

In an embodiment of the composition according to the invention (i) is an adapter CAR cell (AdCAR cell), and (ii) is a radio-labelled ligand of the AdCAR cell, or a radio-labeled ligand for the antigen binding site of the AdCAR cell.

With this measure, the invention takes advantage of the above-mentioned advantages of the adapter CAR cell system. The use of an adapter makes the CAR cell system more flexible - the antigen can be changed in case of resistance, safer-the AdCAR cells are effective only in the presence of the adapter, and multiple antigens can be addressed simultaneously.

In a further embodiment of the AdCAR-comprising composition according to the invention the radio-labelled ligand is selected from the group consisting of: biotin, avidin, fluorescein isothiocyanate (FITC), 10 aa peptide epitope E5B9 (human La/SS-B), 14 aa peptide epitope PNE (yeast GNC4), leucine zipper moiety, SpyTag, $\alpha$-FR$\alpha$, $\alpha$-SAR$\alpha$, and CD19ECD.

Such ligands have been shown to be suitable for the preparation of adapter structures. They are summarized in the publication by Arndt et al. (op. cit.). According to this embodiment, the listed ligands are radio-labeled, e.g., $^{18}$F-biotin, $^{64}$Cu-biotin, $^{18}$F-biotin, $^{64}$Cu-FITC, etc.

In such embodiments the antigen-binding domain of the extracellular domain of the corresponding CAR or AdCAR might be an antibody or an antigen binding fragment that specifically bind to the respective ligand. Such antigen-binding domain include, therefore, anti-biotin antibody or anti-biotin antibody fragment, anti-avidin or anti-avidin fragment, anti-FITC or anti-FITC antibody fragment, etc.....

The CAR or AdCAR antigen-binding domain is typically derived from the variable regions of a monoclonal antibody linked together as a single-chain variable fragment (scFv). An scFv is a chimeric protein made up of the light ($V_L$) and heavy ($V_H$) chains of immunoglobins, connected with a short linker peptide. These $V_L$ and $V_H$ regions are selected in advance for their binding ability to the target antigen (such as biotin, FITC, CD19, etc.). The linker between the two chains consists of hydrophilic residues with stretches of glycine and serine in it for flexibility as well as stretches of glutamate and lysine for added solubility. Single domain antibodies (e.g. $V_H$, $V_H H$) have been engineered and developed as antigen recognition domains in the CAR format due to their high transduction efficiency in immune cells, such as T cells.

The term "antibody" as used herein is used in the broadest sense to cover the various forms of antibody structures including but not being limited to monoclonal and polyclonal antibodies (including full length antibodies), multispecific antibodies (e.g., bispecific antibodies), antibody fragments, i.e., antigen binding fragments of an antibody, immunoadhesins and antibody-immunoadhesin chimeras, that specifically recognize (i.e., bind) a target antigen. "Antibody fragments" comprise a portion of a full length antibody, preferably the variable domain thereof, or at least the antigen binding site thereof ("an antigen binding fragment of an antibody"). Examples of antibody fragments include Fab (fragment antigen binding), scFv (single chain fragment variable), single domain antibodies, diabodies, dsFv, Fab', diabodies, single-chain antibody molecules, and multi-specific antibodies formed from antibody fragments.

As used herein, the term "antigen" is intended to include substances that bind to or evoke the production of one or more antibodies and may comprise, but is not limited to, proteins, peptides, polypeptides, oligopeptides, lipids, carbohydrates, and combinations thereof, for example a glycosylated protein or a glycolipid. The term "antigen" as used herein refers to a molecular entity that may be expressed on a target cell and that can be recognized by means of the adaptive immune system including but not restricted to antibodies or TCRs, or engineered molecules including but not restricted to transgenic TCRs, CARs, scFvs or multimers thereof, Fab-fragments or multimers thereof, antibodies or multimers thereof, single chain antibodies or multimers thereof, or any other molecule that can execute binding to a structure with high affinity.

The CAR or AdCAR antigen-binding domain might also or alternatively comprise non-antibody structures which specifically bind to the respective ligands, such as avidin, leucine zipper moieties, SpyCatchers, folate receptor, EGFRvIII, Cripto-1, etc.; cf. Arndt et al. (op. cit.).

In one embodiment of the invention the composition, therefore, the latter comprises (i) an AdCAR cell expressing an anti-biotin AdCAR, and (ii) a radio-labelled biotin.

In still another embodiment of the AdCAR comprising composition according to the invention the ligand is biotin$_3$.

Biotin$_3$ comprises and preferably consists of 3 linked biotin molecules, and it turned out to have excellent binding capacities. It is assumed by the inventors, without being bound to the theory, that three biotin molecules can bind to three AdCAR surface receptors, thus the avidity is increased, but affinity stays the same. To further optimize the avidity and pharmacokinetic properties more or less biotin molecules could be added.

In yet another embodiment of the invention the radio label comprises a radioisotope having a half-live of at least 20 minutes. Suitable radioisotopes are selected from the group consisting of: $^{18}F$, $^{64}Cu$, $^{68}Ga$, $^{99m}Tc$, $^{89}Zr$, $^{11}C$, $^{124}I$, $^{123}I$, $^{131}I$, $^{177}Lu$, $^{90}Y$, $^{225}Ac$, and $^{211}At$.

This measure has the advantage that suitable radioisotopes, which are particularly proven in the practice of radiodiagnostics and therapy, are used. On the one hand, the short half-life ensures that tracking of the cells is possible. On the other hand, damage to biological healthy tissue can be prevented. However, it is possible to adjust the radiolabeling so that the target or tumor cells are specifically killed with it.

With regard to what was previously mentioned, one preferred radio-labelled ligand of the CAR/AdCAR is [$^{18}F$]biotin$_3$fluorobenzoate or [$^{18}F$]biotin$_3$FB, and, preferably, the corresponding CAR/AdCAR is an anti-biotin CAR/AdCAR or an avidin CAR/AdCAR, respectively.

In a further development of the invention the composition further comprises (iii) an adapter molecule comprising a first moiety and a second moiety.

This measure advantageously brings the composition according to the invention to a large extent already in a ready-to-use state, because the AdCAR cells are only effective in the presence of an adapter.

First and second moiety represent the two generally opposite functional sections of the adapter. In one embodiment of the invention, these can be supplemented by a linker moiety which conjugates the first moiety with the second moiety. The design, configuration and manufacture of an adapter molecule for AdCAR platforms is well known in the art, see, e.g., WO 2018/078066.

In an embodiment of the invention the first moiety comprises a ligand for the chimeric antigen receptor (CAR) of the AdCAR cell, and in another embodiment of the invention the second moiety comprises an antigen binding structure.

With this measure, the constructive conditions are created in an advantageous way to provide a functional adapter. The first moiety of the adapter provides the binding structure or antigen for the CAR/AdCAR or its antigen-binding domain, respectively. The second moiety of the adapter then establishes the specific binding to the target structure, e.g., a surface marker of a tumor cell, such as CD19. The antigen-binding structure of the adapter can preferably be configured in the same way as the antigen-binding domain of the CAR, i.e., comprises an antibody or an antibody fragment (e.g., a scFv) that is specifically directed against the antigen, e.g., a surface marker of a tumor cell, such as CD19.

In still another embodiment of the composition according to the invention the antigen the antigen-binding structure/domain specifically binds to is a tumor cell-specific biomarker, preferably a surface molecule of a tumor cell.

This measure has the advantage that the invention is adapted to the application which CAR cell therapies are best suited for. For example, CAR/AdCAR cells are particularly good at recognizing surface structures. Tumor cells that can be characterized by typical surface markers are therefore preferred targets of the composition according to the invention.

In yet another embodiment of the invention the surface molecule that can be specifically recognized and bound by the composition or CAR/AdCAR cell according to the invention is selected from the group consisting of: CD19, CD22, BCMA, GD2, CD33 (Siglec-3), CD123 (IL3RA), CD135 (FLT-3), CD44 (HCAM), CD44V6, CD47, CD184 (CXCR4), CLEC12A (CLL1), LeY, FRB, MICA/B, CD305 (LAIR-I), CD366 (TIM-3), CD96 (TACTILE), CD133, CD56, CD29 (ITGBI), CD44 (HCAM), CD47 (IAP), CD66 (CEA), CD112 (Nectin2), CD117 (c-Kit), CD133, CD146 (MCAM), CD155 (PVR), CD171 (L1CAM), CD221 (IGF1), CD227 (MUC1), CD243 (MRD1), CD246 (ALK), CD271 (LNGFR), CD20, and EGFR.

By this measure, the composition according to the invention is advantageously configured for addressing key tumor cell surface markers.

In yet another embodiment the antigen binding structure of the second moiety of the adapter is an antibody or the antigen-binding fragment thereof.

The antigen binding domain may comprise, for example, full length heavy chain, Fab fragments, single chain Fv (scFv) fragments, divalent single chain antibodies or diabodies. Any molecule that binds specifically to a given antigen such as antibodies or ligand binding domains from naturally occurring receptors may be used as an antigen binding domain.

In another embodiment the composition is a pharmaceutical composition further comprising a pharmaceutically acceptable excipient.

The pharmaceutical composition may comprise the CAR/AdCAR cells and the radio-labelled ligand and, if applicable, the adapter, in "effective amounts". An "effective amount" (or, "therapeutically effective amount") is an amount sufficient to effect a beneficial or desired clinical result upon treatment. An effective amount can be administered to a subject or living being in one or more doses. In terms of treatment, an effective amount is an amount that is sufficient to palliate, ameliorate, stabilize, reverse or slow the progression of the disease, or otherwise reduce the pathological consequences of the disease. The effective amount is generally determined by the physician on a case-by-case basis and is within the skill of one in the art. Several factors are typically considered when determining an appropriate dosage to achieve an effective amount. These factors include age, sex and weight of the subject, the condition being treated, the severity of the condition and the form and effective concentration of the immune cells administered. For adoptive immunotherapy using antigen-specific T cells, cell doses in the range of about $10^6$-$10^{10}$ (e.g., about $10^9$) are typically infused. Upon administration of the presently disclosed immune cells into the host and subsequent differentiation, T cells that are specifically directed against the specific antigen expand. The immune cells can be administered by any method known in the art including, but not limited to, intravenous, subcutaneous, intranodal, intratumoral, intrathecal, intrapleural, intraperitoneal and directly to the thymus.

Pharmaceutical compositions comprising the presently disclosed CAR or AdCAR cells and the radio-labelled ligand can be conveniently provided as sterile liquid preparations, e.g., isotonic aqueous solutions, suspensions, emulsions, dispersions, or viscous compositions, which may be buffered to a selected pH. Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyol (for example, glycerol, propylene glycol, liquid polyethylene glycol, and the like) and suitable mixtures thereof.

Sterile injectable solutions can be prepared by incorporating the modified CAR or AdCAR cells and the radio-labelled ligand according to the invention in the required amount of the appropriate solvent with various amounts of the other ingredients, as desired. Such compositions may be in admixture with a suitable carrier, diluent, or excipient such as sterile water, physiological saline, glucose, dextrose, or the like. The compositions can also be lyophilized. The compositions can contain auxiliary substances such as wetting, dispersing, or emulsifying agents (e.g., methylcellulose), pH buffering agents, gelling or viscosity enhancing additives, preservatives, flavoring agents, colors, and the like, depending upon the route of administration and the preparation desired. Standard texts, such as "Remington - The Science and Practice of Pharmacy", 23rd edition, 2020, may be consulted to prepare suitable preparations, without undue experimentation.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. Prolonged absorption of the injectable pharmaceutical form can be brought about by the use of agents delaying absorption, for example, aluminum monostearate and gelatin. According to the presently disclosed composition, however, any vehicle, diluent, or additive used would have to be compatible with the modified immune cells or their progenitors. The compositions can be isotonic, i.e., they can have the same osmotic pressure as blood and lacrimal fluid. The desired isotonicity of the compositions may be accomplished using sodium chloride, or other pharmaceutically acceptable agents such as dextrose, boric acid, sodium tartrate, propylene glycol or other inorganic or organic solutes. Sodium chloride can be particularly for buffers containing sodium ions.

The skilled artisan can readily determine the amount of cells and optional additives, vehicles, and/or carrier in compositions and to be administered in methods. Typically, any additives (in addition to the active cell(s) and/or agent(s)) are present in an amount of 0.001 to 50% (weight) solution in phosphate buffered saline, and the active ingredient is present in the order of micrograms to milligrams, such as about 0.0001 to about 5 wt %, about 0.0001 to about 1 wt %, about 0.0001 to about 0.05 wt% or about 0.001 to about 20 wt %, about 0.01 to about 10 wt %, or about 0.05 to about 5 wt %. For any composition to be administered to an animal or human, the followings can be determined: toxicity such as by determining the lethal dose (LD) and LD50 in a suitable animal model e.g., rodent such as mouse; the dosage of the composition(s), concentration of components therein and timing of administering the composition(s), which elicit a suitable response. Such determinations do not require undue experimentation from the knowledge of the skilled artisan, this disclosure and the documents cited herein. And, the time for sequential administrations can be ascertained without undue experimentation.

In another embodiment of the composition according to the invention the CAR cell is and CAR immune cell, preferably selected from the group consisting of CAR T cell, CAR NK cell, CAR NKT cell, CAR Treg cell, and CAR stem cell.

This measure advantageously uses the cells that can express the CAR, are most suitable and proven in the context of CAR cell therapies.

The term "immune cell" refers to a cell that may be part of the immune system and executes a particular effector function such as alpha-beta T cells, NK cells, NKT cells, B cells, innate lymphoid cells (ILC), cytokine induced killer (CIK) cells, lymphokine activated killer (LAK) cells, gamma-delta T cells, mesenchymal stem cells or mesenchymal stromal cells (MSC), monocytes or macrophages. Preferred immune cells are cells with cytotoxic effector function such as alpha-beta T cells, NK cells, NKT cells, ILC, CIK cells, LAK cells or gamma-delta T cells. "Effector function" means a specialized function of a cell, e.g., in a T cell an effector function may be cytolytic activity or helper activity including the secretion of cytokines.

Another subject-matter of the invention is a method of treatment of a living being in need for a CAR cell therapy, comprising the administration of the composition according to the invention to the living being, and, optionally, nuclear imaging said composition in said living being.

The features, characteristics and advantages expressively disclosed for the composition according to the invention apply to the method according to the invention correspondingly.

As used herein, the terms "living being" and "subject" can be used interchangeably. They refer to a mammal such as mouse, rat, cow, pig, goat, chicken, dog, monkey or human. Preferentially, the living being is a human. The subject may be a subject suffering from a disorder such as cancer (a patient), but the subject may be also a healthy subject.

The quantity of CAR/AdCAR cells to be administered will vary for the subject being treated. In a one embodiment, between about $10^4$ and about $10^{10}$, between about $10^5$ and about $10^9$, or between about $10^6$ and about $10^8$ of the presently disclosed modified immune cells are administered to a human subject. More effective cells may be administered in even smaller numbers. In certain embodiments, at least about $1 \times 10^8$, about $2 \times 10^8$, about $3 \times 10^8$, about $4 \times 10^8$, or about $5 \times 10^8$ of the presently disclosed modified immune cells are administered to a human subject. In certain embodiments, between about $1 \times 10^7$ and $5 \times 10^8$ of the presently disclosed modified immune cells are administered to a human subject. The precise determination of what would be considered an effective dose may be based on factors individual to each subject, including their size, age, sex, weight, and condition of the particular subject. Dosages can be readily ascertained by those skilled in the art from this disclosure and the knowledge in the art.

In general, and in an embodiment of said method the isolated immune cell being the basis for the preparation of the CAR/AdCAR cells according to the invention was prepared starting from the living being's own immune cells (autologous) or from immune cells of a reference living being (allogeneic).

It is to be understood that the before-mentioned features and those to be mentioned in the following cannot only be used in the combination indicated in the respective case, but also in other combinations or in an isolated manner without departing from the scope of the invention.

The invention is now further explained by means of embodiments resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

The invention is now described and explained in further detail by referring to the following non-limiting examples and figures.

BRIEF DESCRIPTION OF THE FIGURES

Fig. 1:  Molecular structure biotin$_3$ 11. This molecule was used in order to synthesize the radio-PET tracer [$^{18}$F]biotin$_3$FB.

Fig. 2:  Radiosynthesis of the radiolabeled biotin. To perform the radiosynthesis SFB was linked to the precursor.

Fig. 3:  Scheme of AdCAR T cells connected to a tumor cell over the adaptor molecule, according to an embodiment of the invention. Here, the adaptor molecule is a biotinylated antibody. The CAR binds to the biotin, whereas the antibody binds to the surface receptor of the tumor cell. On the top right is a possible radiotracer (here: biotin) also binding to the CAR T cell.

Fig. 4:  Relative values of the three biotin groups with a dilution series of 1/-V10. The logarithm of the concentration is applied against the relative values (1: no binding, 0: complete binding) and was calculated with GraphPad Prism 9.

Fig. 5:  *In vivo* evaluation of [$^{18}$F]biotin$_3$FB. PET and MR images of representative mice with HCT116 AdCAR (A) or HCT116 CTRL (B). Xenografts are enlarged on the right side and highlighted with a white dashed line. The images represent the last 10 minutes of a 1-hour dynamic scan. (C) Ex vivo biodistribution analysis by gamma-counting of [$^{18}$F]biotin$_3$FB (n = 5 for AdCAR and CTRL). (D) Tumor to muscle ratio (TMR) and absolute tumor uptake of AdCAR and CTRL mice imaged with [$^{18}$F]biotin$_3$FB (n = 5 per group).

Fig. 6:  Time activity curves (TACs) from the tumors and selected organs of mice imaged with [$^{18}$F]biotin$_3$FB (n = 5 per group). Regions of interest (ROIs) have been delineated and evaluated with the Inveon software and visualized via GraphPad Prism 9.

Fig. 7:  *In vivo* evaluation of [$^{18}$F]biotin$_3$FB. PET and MR images of representative mice with Jurkat AdCAR (A) or Jurkat CTRL (B). Images were taken after 1 hour resting uptake. The measurement was repeated on day 1, 3 and 7 after cell injection (day 0). (C) Ex vivo biodistribution analysis by gamma-counting of [$^{18}$F]biotin$_3$FB (n = 5 for AdCAR and CTRL). (D) Uptake of [$^{18}$F]biotin$_3$FB into the spleen for Jurkat AdCAR or Jurkat CTRL (n = 5 per group).

EXAMPLE

1. Material and methods

*Precursor synthesis*

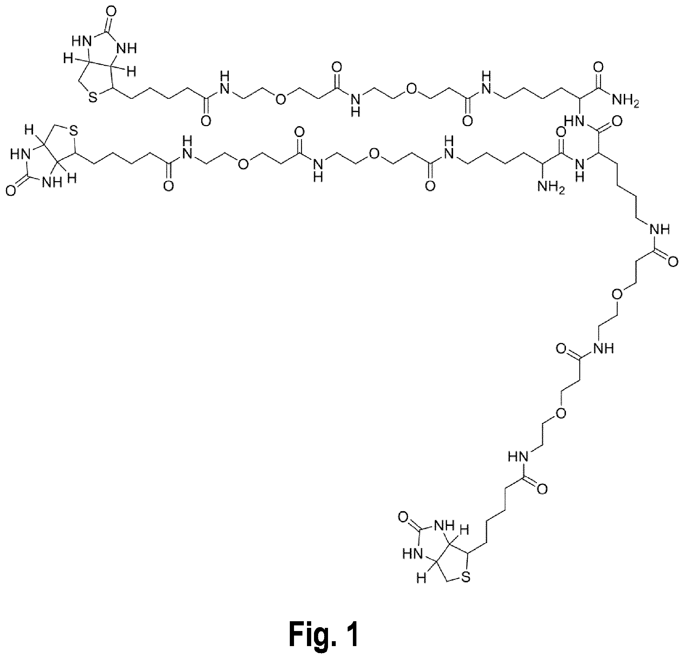

The synthesized precursor was biotin$_3$ 11 (Figure 1). A syringe with frit was filled with Fmoc rink amide resin. 2-3 mL dimethylformamide (DMF) was added and incubated for approximately 10 minutes to let the resin swell. The DMF was discarded and 20% piperidin (solved in DMF) was added to cleave the Fmoc protection group. After 5 minutes of incubation the piperidin was discarded, and the process was repeated. To remove any residues of piperidin the syringe was washed five times for 5 minutes with DMF. For linking the first component Fmoc-L-Lys (Mmt) -OH was dissolved in 2 mL DMF. The solution was transferred into a new reaction vial with 2-(1H-benzotriazole-1-yl)-1,1,3,3-tetramethylaminium tetrafluorborate (TBTU) and dissolved again, using a vortex and ultra-sonic bath. Afterwards, 66 $\mu$L of N-methylmorpholine was added. This mixture was then transferred into the syringe and incubated for one hour on a shaker. After Fmoc cleavage a second Fmoc-L-Lys (Mmt) -OH was coupled as described above. Fmoc was cleaved again and Boc-L-Lys (Fmoc)-OH was coupled, followed by another Fmoc cleavage. The reaction solution was discarded and washed for five times with DMF and five times with isopropanol. The Mmt protection group was cleaved with 1% TFA in DCM and incubated 10 times for one minute. Fmoc-6-amino-4-oxahexanoic acid (OAHX) was dissolved in 2 mL DMF and transferred into a reaction vial with TBTU. After everything was dissolved properly, 66 $\mu$L of NMM was added and the mixture was transferred into the syringe. After one hour of incubation the reaction solution was discarded and a second coupling step with OAHX was performed as described above. For the last coupling, after the washing step, biotin was dissolved in 2 mL N-methyl-2-pyrrolidon (NMP) and transferred into a reaction vial with TBTU. After the mixture was dissolved, 33 $\mu$L NMM were added, and the mixture was transferred into the syringe. The reaction was incubated overnight at room temperature.

The reaction solution was discarded, and the resin was washed five times with isopropanol. Afterwards the syringe was washed five times with diethyl ether (Et$_2$O). To remove possible residues, the syringe was air-dried and incubated for 45 minutes. 2 mL trifluoroacetic acid (TFA) was added and incubated for 90 minutes to cleave the peptide from the resin. One additional mL was added and incubated for another 30 minutes. 50 mL ice-cold Et$_2$O was prepared in a Falcon tube and after incubation, the content of the syringe was added to the ice cold Et$_2$O to precipitate the peptide. This solution was centrifuged for 5 minutes at 5500 rpm. After removing the supernatant another washing step was performed. Afterwards, the precipitate was air dried and dissolved in a mixture of distilled H$_2$O and acetonitrile (max. 3% acetonitrile). It was transferred into a 100 mL round flask and the centrifugation tube was rinsed with water and transferred into the round flask as well. The whole solution was frozen with liquid nitrogen and lyophilized overnight. The resulting product was transferred into a reaction tube and weighed.

*Synthesis of [$^{18}$F]biotin$_3$FB*

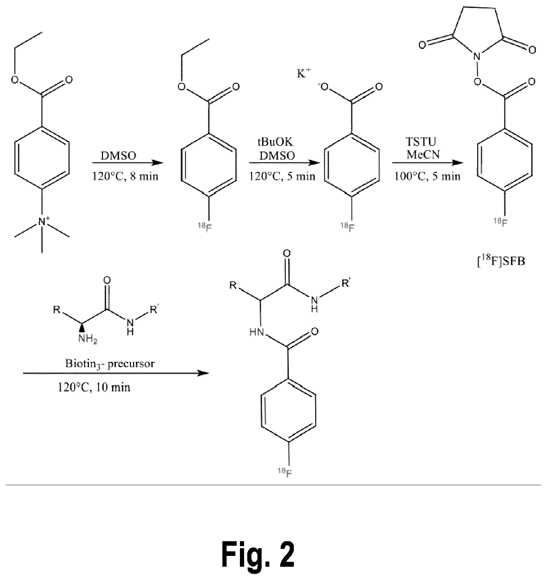

[$^{18}$F]biotin$_3$FB synthesis was automated on a modified TRACERIab radiochemical synthesizer (GE Healthcare, Uppsala, Sweden). The synthesis is illustrated in Figure 2. In brief, [$^{18}$F]fluoride was produced on a medical cyclotron (PETtrace 800, GE Healthcare) using the $^{18}$O(p,n)$^{18}$F nuclear reaction and trapped on an ion exchange cartridge (Sep-Pak Plus Light QMA Carb, Waters, Waltham, MA, USA) preconditioned with 10 ml 1 M NaHCOs and 10 ml H$_2$O. [$^{18}$F]fluoride was eluted with 2 ml 4 % H$_2$O in MeCN containing 9.5 mg Kryptofix 2.2.2 and 1.7 mg K$_2$CO$_3$ and azeotropically dried under a stream of helium. The synthon N-succinimidyl 4-[$^{18}$F]fluorobenzoate ([$^{18}$F]SFB) was synthesized in a one-pot reaction. Successively, 5 mg ethyl-4-trimethylammonium benzoate triflate (SFB precursor) in 0.5 ml dimethylsulfoxide (DMSO) (8 min at 120°C), 15 mg tBuOK in 0.5 ml DMSO (5 min at 120°C) and 30 mg N,N,N',N'-tetramethyl-O-(N-succinimidyl)uronium tetrafluoroborate (TSTU) in 2 ml MeCN (5 min at 100°C) were added to the reaction vial. After cooling to 50°C and addition of 1 ml 5 % acetic acid to prevent hydrolysis, the reaction was diluted in 25 ml H$_2$O and trapped on a Sep-Pak Plus Light C18 cartridge (Waters) preconditioned with 10 ml ethanol and 10 ml H$_2$O. [$^{18}$F]SFB was eluted back in the reactor vial in 0.5 ml DMF containing 10 mg of precursor and 40 $\mu$l DIPEA. The reaction was heated to 120 °C for 10 min. After cooling to 50°C, 2 ml 77% 25 mM ammonium formate/23% MeCN were added, and the reaction mixture was purified on an ABZ+ column (250 mm $\times$ 10 mm, 5 $\mu$m) with 77% 25 mM ammonium formate/23% MeCN and a flow of 6 ml/min. The product peak (retention time 10 min) detected, using an online radioactivity detector) was diluted in 50 ml $H_2O$ and trapped on a Sep-Pak Plus Light C18 cartridge (Waters) preconditioned with 10 ml ethanol and 10 ml $H_2O$. The product was eluted into the product vial with 0.5 ml ethanol, followed by 4.5 ml phosphate-buffered saline (PBS).

*Radio-traceable adapter chimeric antigen receptor cell*

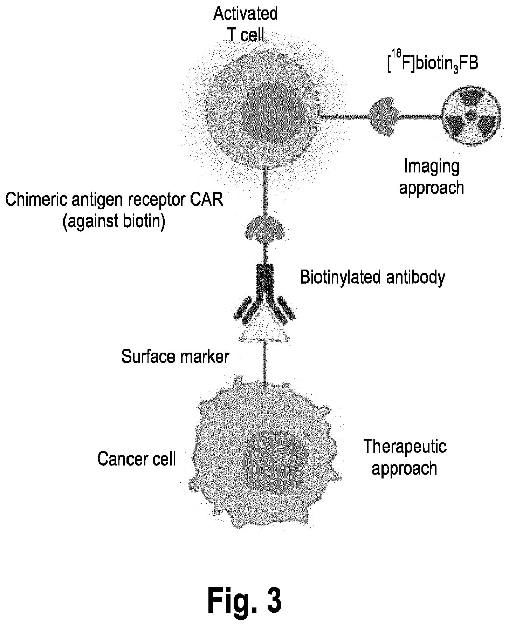

Figure 3 shows a scheme of AdCAR T cells connected to a tumor cell over the adaptor molecule, according to an embodiment of the invention. Here, the adaptor molecule is a biotinylated antibody. The CAR binds to the biotin, whereas the antibody binds to the surface receptor of the tumor cell. On the top right is a possible radiotracer (here: [18F]biotin) also binding to the CAR T cell. Biotin is a small molecule also known as Vitamin B7 and can easily be conjugated to antibodies. Together with engineered AdCAR cells expressing an anti-biotin receptor, they can function for either therapeutic or diagnostic purpose (Figure 3). Since biotin is a comparably small molecule, it is also unlikely to trigger unwanted immune response.

*Cell culture*

HCT116 WT and HCT116 aCAR cells were cultured in a T175 cell culture flask with McCoy's medium supplemented with 10% fetal calf serum (FCS) and 1% Penicillin/Streptomycin. For the second part of the experiment, Jurkat LucGFP and Jurkat aCAR were cultured in T175 Suspension flasks with RPMI medium supplemented with 10% fetal calf serum (FCS) and 1% Penicillin/Streptomycin. The cells were passaged approximately every three days or if they reached a confluency of 90%.

*Cell harvesting*

For the HCT116 cells, the medium was removed, and the cells were washed with 5 mL phosphate-buffered saline (PBS) to remove remaining medium. The PBS was discarded, and 3 mL trypsin were added. The cell culture flask was incubated at 37°C for 5 minutes to allow the cells to detach from the flask surface. 7 mL McCoy's medium was added to stop the reaction and the cell suspension was transferred into a 50 mL centrifugation tube. The cells were centrifuged at 1500 rpm for 5 minutes and 4°C. The Jurkat cells were centrifuged, the medium was removed, and the cells were resuspended in PBS to wash them. Afterwards, the cells were centrifuged again and set to the desired concentration.

*Cell counting*

The pellet resulting from cell harvesting was dissolved in PBS and resuspended thoroughly. 10 $\mu$L of the cell solution was mixed with 90 $\mu$L trypan-blue to create a 1:10 dilution. 10 $\mu$L of the dilution were added into a C-Chip chamber (NanoEntek) and the cells in 4 fields were counted via microscope. To determine the cell concentration following equation was used.

$$c = \frac{cells\ counted}{4} \cdot 10 \cdot 10^4 \qquad\qquad eq.\,1$$

*Tumor inoculation*

First, a tumor growth study was performed to determine the optimal cell concentration. Out of the three possibilities $1 \times 10^6$, $5 \times 10^6$ and $1 \times 10^7$ *cells/mL* the most homogenous growth was achieved using $5 \times 10^6$ *cells/mL.*
For the xenograft model, $5 \times 10^6$ *cells/mL* cells were injected. Five NOD.CB17-Prkdcscid/J were injected with HCT116 WT, and five additional mice were injected with HCT116 AdCAR subcutaneously into the right flank in a mixture of 1:1 Matrigel and PBS (200 $\mu$L per mouse) with a 1 mL syringe (30G canula). Afterwards the tumors grew for approximately two weeks before PET and MR measurements.
Five mice were injected with $1 \times 10^7$ *cells/mL* Jurkat LucGFP (CTRL), and five additional mice were injected with $1 \times 10^7$ *cells/mL* Jurkat aCAR in 200$\mu$l PBS into the peritoneal cavity with a 1 mL syringe (30G canula).

*PET and MR measurements*

Xenograft model

For the xenograft model a one-hour dynamic PET scan was performed. The mice were anaesthetized with isoflurane (initially 5%, then 1.5-2%) and a catheter was placed into the tail vein. The mice were placed onto the scanner bed and the body was centered into the field of view (FOV). Mice were injected with13.6 $\pm$ 0.35 MBq of [$^{18}$F]biotin$_3$FB on average at the beginning of the PET scan and measured for 1h. Afterwards, MR scans were performed to gain anatomical information using an TurboRare spin-echo sequence. After the scans, the mice were sacrificed, and the organs were collected to quantify radiotracer uptake by ex *vivo* gamma-counting.

Jurkat model

For the Jurkat model a ten-minute static PET scan was performed. The mice were anaesthetized with isoflurane (initially 5%, then 1.5-2) and a catheter was placed into the tail vein. The mice were injected with 13.6 $\pm$ 0.24 MBq of [$^{18}$F]biotin$_3$FB on average and afterwards placed in an isoflurane box for a one-hour resting uptake. After 1 h, the mice were placed onto the scanner bed, the body was centered into the FOV, and a 10 min PET scan was performed. Afterwards, MR scans were performed as described above. The PET and MR measurements took place on day 1, 3 and 7 after cell injection. When the measurements on the 7th day were finished, the mice were sacrificed and uptake in spleen, lymph nodes, muscle and blood were quantified by ex *vivo* gamma-counting.

*PET data analysis*

Reconstruction and analysis of the acquired PET/MR data was performed with Inveon Acquisition Workplace and Inveon Research Workplace (IRW), respectively, using dynamic framing and an ordered subset expectation maximization (OSEM) 3D algorithm. For the second part of the experiment statistic framing was used. Regions of interest (ROIs) were drawn under consideration of the acquired MR images and co-registered with the PET data to obtain time-activity curves (TACs).

*Statistical analysis*

Statistical analyses are represented as mean value $\pm$ standard deviation. Analyses were performed in GraphPad Prism 9 using nonparametric and parametric t-tests. p-values < 0.05 were considered statistically significant (*:p-value < 0.05; **:p-value < 0.01; **:p-value <0.001; ****:p-value < 0.0001).

2. Results

The inhibitory capacities of 12 different biotin variants were evaluated to determine the best molecule to transition to in vivo studies. The biotin molecules differed in the number of biotin (one, two or three: biotin,, biotin$_2$ or biotin$_3$) and the number of oxygens within the linker molecules. As linker molecules, either two AHX, two OAHX, or one of each was used to vary hydrophilicity (0 = AHX; 1 = OAHX; e.g., biotin$_3$ 01 consists of three biotin molecules with first AHX and second OAHX as linker).

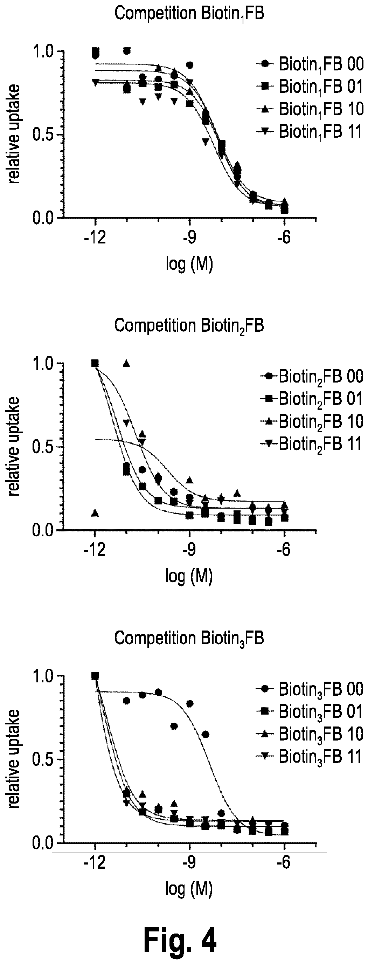

To determine the best biotin radiotracer, an *in vitro* competition assay was performed as part of the project module. Figure 4 shows the results of a competition assay leading to the decision of biotin$_3$FB as most promising radiotracer for further *in vivo* studies. With an IC$_{50}$ of 0.5837 pM it shows great inhibitory capacities at comparatively low concentrations.

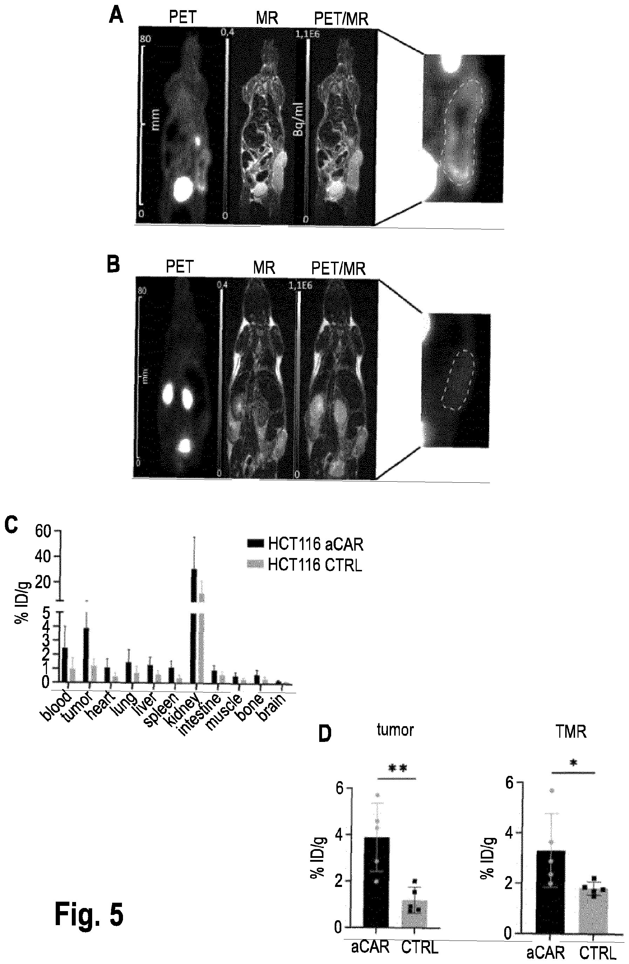

[$^{18}$F]biotin$_3$FB was synthesized in 81 min with high specific activity (239 $\pm$ 177 GBq/$\mu$mol), decay-corrected yield (1.5 $\pm$ 1.7%) and good radiochemical purity (>95%) (n = 5). In the PET and MR images, the HCT116 AdCAR xenografts are clearly distinguishable from the control group xenografts (Fig. 5 A and B). This was further confirmed in the *ex vivo* biodistribution analysis (Fig. 5 C) where the AdCAR expressing xenografts show a higher uptake than the control xenografts (AdCAR = 4.59% ID/g, CTRL = 0.73% ID/g). High radiotracer uptake was also observed in the kidney, pointing towards mainly renal clearance, and the blood. Almost no uptake was observed in the brain, indicating that the radiotracer might not be able to cross the blood-brain barrier. The absolute tumor uptake was significantly higher (p = 0.0050; unpaired t test) in the AdCAR xenografts compared to control xenografts, but as uptake was generally higher in the group carrying the AdCAR xenografts, these results should be interpreted with caution. A more neutral quantification is the tumor-to-muscle ratio, where uptake is quantified in relation to a reference tissue (e.g., the muscle). Indeed, also the TMR was significantly higher in the AdCAR xenografts compared to control xenografts (P=0.0159; Mann-Whitney test).

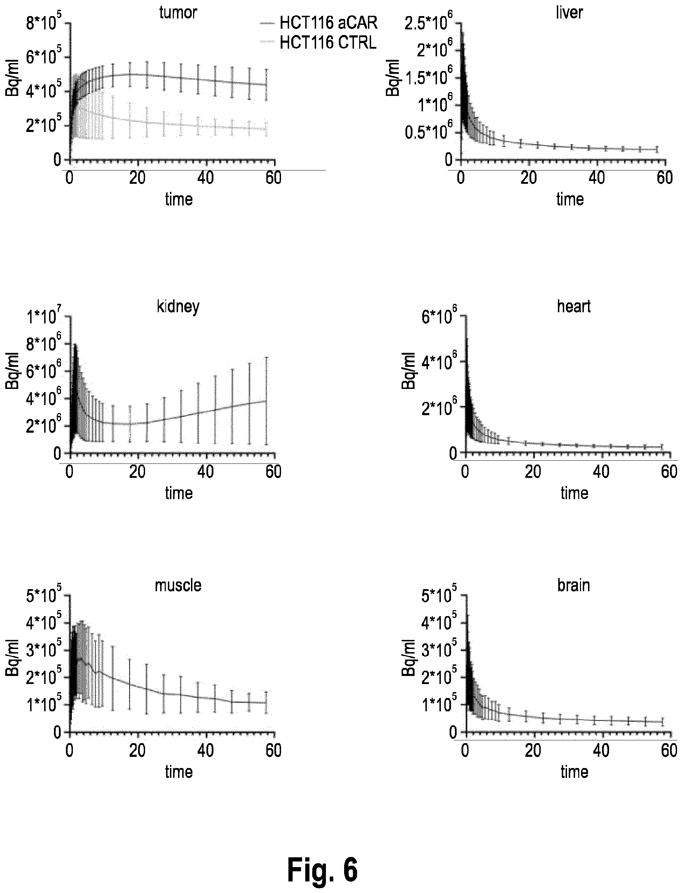

The time activity curves (TACs) obtained from ROIs drawn in the MR images show higher radiotracer uptake for AdCAR xenografts over time compared to control xenografts (Fig. 6). Although the initial uptake is high, the liver TAC decreases rapidly to only background. In contrast, the kidney TAC increases over time, confirming the hypothesis of mainly renal metabolism. Fast decrease in the heart TAC demonstrates fast blood clearance, further confirmed by the calculated blood half-life of 2.3 min. As expected from the biodistribution, the brain TACs show only low uptake (SUVpeak(aCAR) = 0.54; SUVpeak(CTRL) = 0.45).

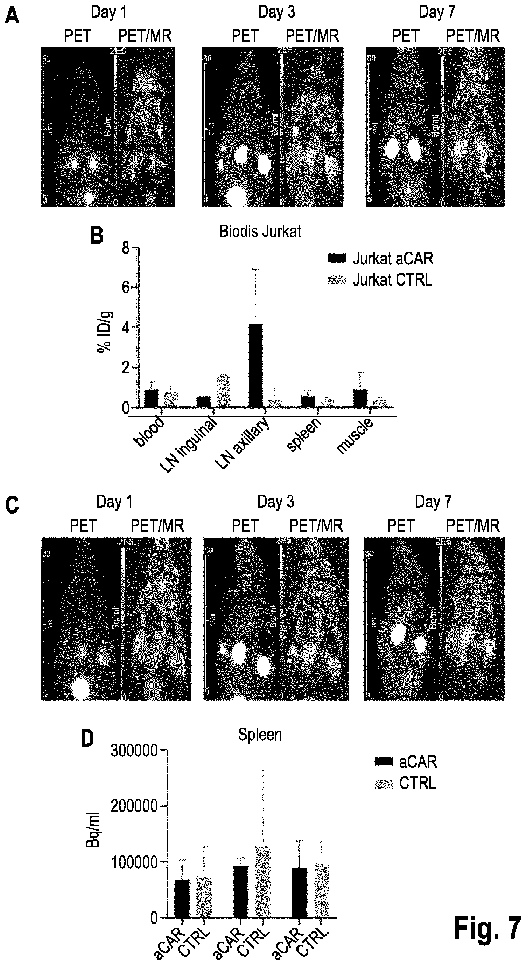

In the Jurkat model, no difference was observed between the AdCAR expressing cells and the control cells at all timepoints (Figure 7 A/B). Most radioactive signal was observed in kidneys, as this is the main excretory route, and the bladder. However, both models also show a slight uptake on the left side of the intestine region. In the *ex vivo* biodistribution, no significant difference was observed in the spleen, inguinal and axillary lymph nodes, muscle, and blood (Figure 7C). The radiotracer uptake in the axillary lymph nodes seem to be increased, however, this was not statistically significant as the variance was very high due to experimental reasons (axillary lymph nodes were not found in some of the mice). Additionally, spleen uptake was quantified with IRW *ex vivo* since it was expected that T cells home in the spleen. However, both models have a similar radiotracer uptake into the spleen and no difference was observed between the AdCAR model and the controls at all time points (Figure 7D). The organs were harvested for histological detection of the Jurkat cells.

3. Summary and interpretation of the experiments

Reporter-free tracking of immune cells would be of great benefit for monitoring AdCAR T cell therapy success. Radiolabeled biotin is a promising first exemplary tool to achieve this goal. The inventors were able to demonstrate that the biotin$_3$FB shows the best binding capacities (Figure 4). The inventors were in the following able to show that the radiotracer is suitable for *in vivo* imaging and CAR T cell tracking.

A total of 12 biotin molecules were tested in terms of hydrophobicity, binding, and size. The most efficient variant, [$^{18}$F]biotin$_3$FB, consists of 3 linked biotin molecules and was then transferred to *in vivo* experiments. For the *in vivo* study NOD.CB17-Prkdcscid/J mice were subcutaneously injected with either control or anti-biotin AdCAR expressing HCT116 cells. After xenograft growth, the mice underwent dynamic PET and MR imaging, and the organs were collected to quantify radiotracer distribution by gamma-counting. In the following, an immortalized T cell line (Jurkat) carrying either the AdCAR or LucGFP as size control was intraperitoneally injected to determine that cell tracking using [$^{18}$F]biotin$_3$FB is possible.

The reason for biotin$_3$ showing the best binding capacities is because three biotin molecules can bind to three AdCAR surface receptors, thus the avidity is increased, but affinity stays the same. To further optimize the avidity and pharmacokinetic properties more or less biotin molecules could be added.

The AdCAR xenograft shows clear radioactive signal on the tumor surface (Figure 5A). Less signal was observed towards in the center of the tumor, pointing either to loss of AdCAR expression or beginning necrosis of the tumor.

The control group shows only background signal of the radiotracer in the tumor region (Figure 5B). This confirms that the binding is specific to the AdCAR xenografts, and thus confirms the general ability of [$^{18}$F]biotin$_3$FB to target anti-biotin AdCAR. This conclusion is further confirmed by the absolute tumor value and the TMR (Figure 5D), showing significantly higher uptake in the AdCAR model. Both groups show high uptake within the urinary bladder and kidneys but low uptake in liver tissue, pointing towards mainly renal clearance of [$^{18}$F]biotin$_3$FB. This hints towards suitability of the radiotracer for also difficult-to-reach liver metastasis, as hepatobiliary excretion hampers the use of many radiotracers due to high background uptake. Under natural conditions biotin is added to the organism over food like yeast, green-leaf vegetables, egg, etc., in form of biotinylated peptides or biocytin. By facilitating a catalytic reaction through biotinidase it is released and can be transported across the cell membrane via sodium-dependent transporters. Here it acts as co-factor for a variety of katabolic reactions. The radiotracer of the inventors is very different from the natural occurring since it consists of multiple linkers and biotin molecules. Thus, the background of [$^{18}$F]biotin$_3$FB in non-target tissue is comparably low and distributed evenly.

The TACs show an initial intermediate activity for both tumor models at the beginning, but while the AdCAR xenografts rise over time, the CTRL xenografts remain at a low level, confirming the findings from the *ex vivo* biodistribution (Figure 6). Furthermore, high kidney and low liver uptake over time was verified, although the liver shows an initial high uptake. The muscle TAC shows only background signal, which confirms its use as reference tissue for the TMR. No uptake was observed in the brain, thus the radiotracer is not able to cross the blood-brain barrier (SUVpeak(aCAR) = 0.54; SUVpeak (CTRL) = 0.45). Taken together, [$^{18}$F]biotin$_3$FB demonstrated good and specific binding to AdCAR expressing cells and shows a favorable pharmacokinetic profile for translation into clinical use.

For the Jurkat model, no significant difference between the AdCAR expressing cells and the control cells was observed. A higher uptake in the spleen of mice with the Jurkat AdCAR model was expected as T cells home to lymphoid organs of which the spleen is the biggest. Neither the PET and MR evaluation (Figure 7 A/B), biodistribution (Figure 7 C) nor ROI quantification of the spleen (Figure 7 D) have shown any significant difference, however, high kidney and bladder uptake further confirmed the biodistribution data from the xenograft model. Reason for this could be the comparably low cell concentration, as only $1 \times 10^7$ *cells/mL* were injected compared to a much higher cell density in the xenografts over the growth of two weeks. So, it is likely, that the AdCAR concentration simply was not sufficient. In addition, T cells experience a rather fast degradation which could lead to low tracer uptake as well.

In short, [$^{18}$F]biotin$_3$FB showed excellent binding towards the AdCAR expressing tumor cells *in vitro* and in the *in vivo* xenograft study. The AdCAR xenografts were clearly distinguishable from the surrounding tissue in contrast to the CTRL group. [$^{18}$F]biotin$_3$FB shows promising pharmacokinetic properties that need to be evaluated further, for example *in vivo* metabolite analyses to exclude any effects from radiometabolites.

The general inventive concept can be extended to different AdCAR constructs, e.g., anti-GD2 CARs using a ganglioside located on the surface of tumors like neuroblastoma and melanoma.

The use of for reporter-free imaging of AdCAR T cell therapy according to the invention is a rewarding approach for immunotherapy since it comes with several advantages. Once the AdCAR is engineered there are multiple areas of application. As T cell therapies in general show only low efficacy in solid tumors, AdCAR T cell imaging could aid shedding light on the mechanistic behind T cell penetration. In addition, there are no risks regarding immunogenicity, which makes it more suitable for patients.

With the invention AdCAR T cell therapy will be more controllable and predictable. This will significantly aid patient care and be a big step towards personalized cancer treatment in the future.

## Claims

1. A composition comprising (i) a chimeric antigen receptor cell (CAR cell), and (ii) a radio-labelled ligand for the chimeric antigen receptor (CAR) of the CAR cell.

2. The composition of claim 1, wherein (ii) is a radio-labelled ligand for the antigen binding site of the CAR of the CAR cell.

3. The composition of claim 1 or 2, wherein (i) is an adapter CAR cell (AdCAR cell), and (ii) is a radio-labelled ligand of the AdCAR cell, or a radio-labeled ligand for the antigen binding site of the AdCAR cell.

4. The composition of claim 2 or 3, wherein the radio-labelled ligand is selected from the group consisting of: biotin, avidin, fluorescein isothiocyanate (FITC), 10 aa peptide epitope E5B9 (human La/SS-B), 14 aa peptide epitope PNE (yeast GNC4), leucine zipper moiety, SpyTag, $\alpha$-FR$\alpha$, $\alpha$-SAR$\alpha$, and CD19ECD.

5. The composition of claim 3 or 4, wherein the ligand is biotin$_3$.

6. The composition of any of the preceding claims, wherein the radio label comprises a radioisotope having a half-live of at least 20 minutes.

7. The composition of claim 6, wherein the radioisotope is selected from the group consisting of: $^{18}$F, $^{64}$Cu, $^{68}$Ga, $^{99m}$Tc, $^{89}$Zr, $^{11}$C, $^{124}$I, $^{123}$I, $^{131}$I, $^{177}$Lu, $^{90}$Y, $^{225}$Ac, and 211At.

8. The composition of any of the claims 2-7, further comprising (iii) an adapter molecule comprising a first moiety and a second moiety.

9. The composition of claim 8, wherein the first moiety comprises a ligand for the chimeric antigen receptor (CAR) of the AdCAR cell.

10. The composition of claim 8 or 9, wherein the second moiety comprises an antigen binding structure.

11. The composition of claim 10, wherein the antigen is a tumor cell-specific biomarker.

12. The composition of claim 10 or 11, wherein the antigen is a surface molecule of a tumor cell.

13. The composition of claim 12, wherein the surface molecule is selected from the group consisting of: CD19, CD22, BCMA, GD2, CD33 (Siglec-3), CD123 (IL3RA), CD135 (FLT-3), CD44 (HCAM), CD44V6, CD47, CD184 (CXCR4), CLEC12A (CLL1), LeY, FRB, MICA/B, CD305 (LAIR-I), CD366 (TIM-3), CD96 (TACTILE), CD133, CD56, CD29 (ITGBI), CD44 (HCAM), CD47 (IAP), CD66 (CEA), CD112 (Nectin2), CD117 (c-Kit), CD133, CD146 (MCAM), CD155 (PVR), CD171 (L1CAM), CD221 (IGF1), CD227 (MUC1), CD243 (MRD1), CD246 (ALK), CD271 (LNGFR), CD20, and EGFR.

14. The composition of any of claims 10-13, wherein the antigen binding structure is an antibody or the antigen-binding fragment thereof.

15. The composition of any of the preceding claims, wherein the CAR cell is and CAR immune cell, preferably selected from the group consisting of CAR T cell, CAR NK cell, CAR NKT cell, CAR Treg cell, and CAR stem cell.

## Fig. 1

Fig. 2

Activated
T cell

$[^{18}F]biotin_3FB$

Imaging
approach

Chimeric antigen receptor CAR
(against biotin)

Biotinylated antibody

Surface marker

Cancer cell

Therapeutic
approach

Fig. 3

Fig. 4

Fig. 5

**Fig. 6**

**Fig. 7**

Europäisches
Patentamt

European
Patent Office

Office européen
des brevets

# EUROPEAN SEARCH REPORT

Application Number

EP 23 16 1072

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 106 282 237 A (UNIV BEIJING) 4 January 2017 (2017-01-04) * the whole document * | 1-4,6-15 | INV. A61K39/00 C07K16/00 |
| X | LIU JINGJING ET AL: "Quantitative radio-thin-layer chromatography and positron emission tomography studies for measuring streptavidin transduced chimeric antigen receptor T cells", JOURNAL OF CHROMATOGRAPHY B, ELSEVIER, AMSTERDAM, NL, vol. 1182, 23 September 2021 (2021-09-23), XP086810057, ISSN: 1570-0232, DOI: 10.1016/J.JCHROMB.2021.122944 [retrieved on 2021-09-23] | 1-4,6,7, 13,15 | |
| Y | * the whole document * | 5 | |
| X | IL MINN ET AL: "Imaging CAR T cell therapy with PSMA-targeted positron emission tomography", SCIENCE ADVANCES, vol. 5, no. 7, 1 July 2019 (2019-07-01), page eaaw5096, XP055725588, DOI: 10.1126/sciadv.aaw5096 * the whole document * | 1-3,6,7, 15 | |
| Y | WILBUR D S ET AL: "BIOTIN REAGENTS FOR ANTIBODY PRETARGETING. 3. SYNTHESIS, RADIOIODINATION, AND EVALUATION OF BIOTINYLATED STARBURST DENDRIMERS", BIOCONJUGATE CHEMISTRY, AMERICAN CHEMICAL SOCIETY, US, vol. 9, no. 6, 1 November 1998 (1998-11-01), pages 813-825, XP000786597, ISSN: 1043-1802, DOI: 10.1021/BC980055E * the whole document * | 5 | |

TECHNICAL FIELDS SEARCHED (IPC)

A61K
C07K

The present search report has been drawn up for all claims

| Place of search | Date of completion of the search | Examiner |
|---|---|---|
| The Hague | 29 September 2023 | de Buhr, Hendrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 4 427 759 A1

## ANNEX TO THE EUROPEAN SEARCH REPORT
## ON EUROPEAN PATENT APPLICATION NO.

EP 23 16 1072

This annex lists the patent family members relating to the patent documents cited in the above-mentioned European search report.
The members are as contained in the European Patent Office EDP file on
The European Patent Office is in no way liable for these particulars which are merely given for the purpose of information.

29-09-2023

| Patent document cited in search report | Publication date | Patent family member(s) | Publication date |
|---|---|---|---|
| CN 106282237 A | 04-01-2017 | NONE | |

EPO FORM P0459

For more details about this annex : see Official Journal of the European Patent Office, No. 12/82

24

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018078066 A

**Non-patent literature cited in the description**

- **ARNDT, C. et al.** Adaptor CAR Platforms - Next Generation of T Cell-Based Cancer Immunotherapy. *Cancers,* 2020, vol. 12 (1302
- **SEITZ et al.** Novel adapter CAR-T cell technology for precisely controllable multiplex cancer targeting. *Oncoimmunology,* 2021, vol. 10 (1), e2003532
- **MAROFI, F. et al.** CAR T cells in solid tumors: challenges and opportunities. *Stem Cell Res Ther,* 2021, vol. 12 (81
- **KIM, W. et al.** In vivo tracking of bioorthogonally labeled T-cells for predicting therapeutic efficacy of adoptive T-cell therapy. *J. Control. Release,* 2021, vol. 329, 223-236
- **SELLMYER, M. A. et al.** Imaging CAR T Cell Trafficking with eDHFR as a PET Reporter Gene. *Mol. Ther.,* 2020, vol. 28, 42-51
- Remington - The Science and Practice of Pharmacy. 2020